(19)

Europäisches
Patentamt
European
Patent Office
Office européen
des brevets

(11) **EP 1 504 047 B1**

(12) **EUROPEAN PATENT SPECIFICATION**

(45) Date of publication and mention
of the grant of the patent:
**12.12.2007 Bulletin 2007/50**

(21) Application number: **03717321.8**

(22) Date of filing: **24.04.2003**

(51) Int Cl.:
*C08G 63/06* (2006.01)    *C08G 63/08* (2006.01)
*C08G 63/91* (2006.01)    *C08G 63/58* (2006.01)
*C08G 63/48* (2006.01)    *C08G 65/08* (2006.01)
*C08G 63/64* (2006.01)    *C08L 69/00* (2006.01)
*A61K 47/10* (2006.01)    *A61K 47/34* (2006.01)

(86) International application number:
**PCT/EP2003/004368**

(87) International publication number:
**WO 2003/093344 (13.11.2003 Gazette 2003/46)**

(54) **POLYMERIC MICROEMULSIONS**

POLYMERMIKROEMULSIONEN

MICROEMULSIONS POLYMERES

(84) Designated Contracting States:
**AT BE BG CH CY CZ DE DK EE ES FI FR GB GR
HU IE IT LI LU MC NL PT RO SE SI SK TR**
Designated Extension States:
**AL LT LV MK**

(30) Priority: **03.05.2002 US 377901 P**

(43) Date of publication of application:
**09.02.2005 Bulletin 2005/06**

(73) Proprietor: **Janssen Pharmaceutica NV
2340 Beerse (BE)**

(72) Inventors:
• **Arien, Albertina Maria Eduarda
2340 Beerse (BE)**
• **Brewster, Marcus Eli
2340 Beerse (BE)**
• **Nathan, Aruna
Somerville, NJ 08876 (US)**

• **Rosenblatt, Joel
Somerville, NJ 08876 (US)**
• **Ould-Ouali, Louisa Myriam
1200 Brussels (BE)**
• **Préat, Véronique
1200 Brussels (BE)**

(74) Representative: **Vervoort, Liesbeth
J&J Patent Law Department Beerse
Janssen Pharmaceutica N.V.
Turnhoutseweg 30
2340 Beerse (BE)**

(56) References cited:
**EP-A- 0 258 749       EP-A- 0 258 780
EP-A- 0 952 171       WO-A-02/38184
WO-A-97/45105       WO-A-98/35631
WO-A-99/36100       US-A- 4 716 203
US-B1- 6 211 249       US-B1- 6 221 977
US-B1- 6 322 805**

**Description**

This invention relates to diblock copolymers, compositions comprising said diblock copolymers and an active ingredient, and pharmaceutical dosage forms comprising said compositions for the administration of poorly water soluble drugs.

A microemulsion can be defined according to Danielsson and Lindman (Colloid Surf., 3, 391, 1981) as an optically isotropic and thermodynamically stable transparent to translucent (droplet size of the dispersed phase, typically <140 $\mu$m) liquid system comprising at least the following three components : water (polar phase), oil (apolar phase) and an amphiphilic surfactant, i.e. a surfactant characterized by a hydrophilic and a hydrophobic part. In order to increase the stability of the microemulsion, a cosurfactant may also be present. The surfactant molecules and, when present, also the cosurfactant molecules, arrange themselves at the oil/water interface thereby stabilizing the microemulsion system. In case of pharmaceutical microemulsions, the system contains a further component, i.e. a drug.

From a pharmaceutical point of view, especially oil in water microemulsions have potential to act as drug delivery vehicles. In these oil in water microemulsions, the oil phase is the dispersed (inner) phase and the water phase is the continuous (outer) phase. A wide range of poorly water soluble drug molecules can be incorporated into the apolar, inner oil phase or in the surfactant layer forming the oil-water interphase. In this way the solubility of the drug can be increased when compared to pure water and consequently also the bioavailability of the drug. Water in oil microemulsions, where water is the inner and oil is the outer phase, are less attractive for oral or parenteral administration since the oily phase as continuous, outer phase can give taste problems and because water in oil microemulsions are destabilized to a much greater extent when diluted by an aqueous phase (e.g. upon oral or parenteral administration). Pharmaceutical microemulsions are typically developed for oral, parenteral and topical administration.

A self-microemulsifying drug delivery system (SMEDDS) can be described as an optically isotropic system of oil , surfactant and drug, which forms an oil in water microemulsion on gentle agitation in the presence of water, e.g. in the presence of gastro-intestinal fluids upon oral administration. A SMEDDS for pharmaceutical application can thus be considered as a concentrate which is rapidly dispersed when introduced into the body to form an oil in water microemulsion. An example of a pharmaceutical SMEDDS is Neoral® (Novartis AG, Basel, Switzerland) which is an isotropic blend of surfactant, medium chain length triglyceride oil and cyclosporine A.

Instead of conventional surfactant molecules, amphiphilic diblock copolymers can also be used to form microemulsions. The amphiphilic diblock copolymers arrange themselves at the oil/water interphase whereby the hydrophobic part of the copolymers directs itself to the oil phase while the hydrophilic part directs itself to the water phase. In this way the amphiphilic block copolymers stabilize the microemulsion system in a way that is comparable to conventional surfactants. An advantage of the diblock copolymers over conventional surfactants is the relative ease with which the physicochemical properties can be tailored.

Besides the use in microemulsions, amphiphilic block copolymers can also be used to prepare aqueous micellar solutions. When introduced in water, the copolymers self-associate to form polymeric micelles. These polymeric micelles can be considered as core-shell structures, the inner core being comprised of the hydrophobic part of the block copolymer molecules and the shell or corona being formed by the hydrophilic part of the copolymer molecules. Diverse drugs with a hydrophobic nature can be loaded into the core of the micelles, allowing them to be solubilized in an aqueous medium. In this way, the solubility and bioavailability of poorly water soluble drugs can be enhanced.

The formation of aqueous solutions of drug loaded micelles is not straightforward. The simple addition of drug and amphiphilic block copolymer to water may not result in micelle formation or in a high level of incorporated drug. Complex or time consuming methods are generally used to physically entrap drugs in polymeric micelles. These methods comprise :

> a) stirring : a drug is added to an aqueous solution of an amphiphilc block copolymer and stirred for a substantial period of time in order to load the micelles with drug;
> b) heating : a drug is added to an aqueous solution of an amphiphilic diblock copolymer and stirred at elevated temperatures (e.g. 50 to 120°C) for a certain period of time. The solution is subsequently cooled to room temperature while stirring in order to obtain a drug loaded micellar solution;
> c) ultrasonic treatment : a drug can be loaded into polymeric micelles by ultrasonic treatment of a micellar solution to which the drug is added. After ultrasonic treatment the solution is stirred at room temperature resulting in a micellar solution containing the drug;
> d) solvent evaporation : a drug is dissolved in a volatile organic solvent and added to an aqueous solution of an amphiphilic block copolymer. The organic solvent is subsequently evaporated by stirring the solution. Drug which is not loaded in that way into micelles, can be removed by filtration;
> e) dialysis : drug and block copolymer are dissolved in an organic solvent and the mixture is subsequently dialysed against water. As the organic solvent is gradually replaced by water, the hydrophobic parts of the block copolymer associate to form micellar structures thereby incorporating the drug in the cores. When dialysis is continued for an extended period, complete removal of the organic solvent may be ensured. As an alternative, water may also be added dropwise to a solution of drug and amphiphilic block copolymer in an organic solvent. To remove the organic solvent, the copolymer-drug micellar solution may be finally dialysed against water.

It has now been found that when using the compositions of the present invention, drug loaded micellar solutions with a satisfactory level of drug load can be formed without the need of heat or at relative low temperature, i.e. below 50°C, without the need of organic solvents, complex or time consuming manufacturing processes. This is appealing from an industrial point of view. The polymers/compositions of the invention are self-emusifying, meaning that they spontaneously form upon mild agitation micelles/drug loaded micelles when added to aqueous media. The compositions of the invention can in fact be regarded as polymeric microemulsions, in particular as concentrates of an active ingredient and a diblock copolymer, comparable to a SMEDDS as described hereinabove with the difference that the function of oil and surfactant is now combined in the diblock copolymer.

Since the polymers/compositions of the present invention can give rise to drug loaded micellar solutions, they can be used to increase the solubility and hence the bioavailability of poorly water soluble drugs. This is an important feature from a pharmaceutical point of view. Many drug compounds, while possessing desired therapeutic properties, are used inefficiently due to their poor water solubilities. Thus for example where such compounds are administered orally, only a small fraction of the drug is taken up into the blood during transit of the gastro-intestinal tract. As a result, to achieve adequate drug uptake it may be necessary to administer high doses of the drug compound, to prolong the period of drug administration or to make frequent administrations of the drug compound. Indeed, the poor solubility and hence poor bioavailability of a drug may cause an alternative drug, perhaps one with undesired side effects or one which requires invasive administration (e.g. by injection or infusion), to be used in place of the poorly soluble drug.

Hagan et al. (Langmuir, 12, 2153-2161 (1996)), discloses copolymers of polylactide (PLA) and poly(ethylene glycol) (PEG). These copolymers are described as being directly dispersible in aqueous media. However, to prepare clear aqueous dispersions, the PEG-PLA copolymers are dissolved in water and the resulting copolymer/water system is retained at room temperature for several hours with occasional shaking. Two model drugs were incorporated into said PEG-PLA micellar solutions by adding organic solutions of these drugs to the aqueous dispersions or by sonicating the PEG-PLA/drug dispersions.

EP-B-0,166,596 describes self-dispersible copolymers. The copolymers are rendered self-dispersible by freeze-drying aqueous dispersions of these copolymers. EP-B-0,166,596 also relates to a solid copolymer/drug powder material which is obtained by freeze-drying an aqueous dispersion of the self-dispersible copolymer, obtained as described above, and the drug.

Zhang et al. (Int. J. Pharm. 132, 195-206 (1996)) describes a solid taxol/poly(DL-lactide-co-methoxy polyethylene glycol) (PDLLA-MePEG) matrix obtained by evaporating a solution of taxol and PDLLA-MePEG in acetonitrile. In order to obtain a taxol loaded micellar solution, the solid taxol/PDLLA-MePEG matrix is preheated, followed by adding water at about 60°C and stirring to obtain a clear micellar solution.

Matsuda et al. (Macromolecules (2000), 33, 795-800) discloses liquid biodegradable copolymers of ε-caprolactone and trimethylene carbonate prepared by ring opening polymerization initiated by trimethylene glycol, trimethylolpropane, pentaerythritol or diglycerol poly(ethylene glycol ether). Said copolymers are subsequently derivatized with coumarin at their hydroxyl terminus in order to obtain photocurable coumarin-end-capped biodegradable polymers.

EP-B-0,711,794 relates to injectable liquid copolymers for soft tissue repair and augmentation. The exemplified copolymers are synthesized by a ring opening polymerization reaction with ε-caprolactone, L-lactide, para-dioxanone or trimethylene carbonate and initiated with glycerol, 1-dodecanol or propylene glycol.

EP-B-0,411,545 relates to random copolymers of para-dioxanone, lactide and/or glycolide as coating polymers for surgical filaments. The exemplified copolymers are prepared by heating the initiators, monomers and catalysts for a certain period of time. As initiators diethylene glycol, mannitol, glycerol and glycolic acid are used.

US 6322805 describes hydrophobic drug solution compositions containing polymeric micelles consisting of amphiphilic di -or triblock copolymers.

Viewed from one aspect, the invention provides diblock copolymers consisting of a linear hydrophilic polymer block and a hydrophobic polymer block, said diblock copolymers being liquid at a temperature below 50°C, The copolymers have self-emulsifying properties, they spontaneously form a micellar solution in an aqueous medium upon mild agitation. There is no need for surfactants, extensive heat (temperature below 50°C suffices), organic solvents, complex or time consuming processes to prepare the micellar solutions. Also the preparation of drug loaded micellar solutions from the diblock copolymers of the invention does not require extensive heat, organic solvents, complex or time consuming processes.

In particular, the present invention concerns a diblock copolymer of formula A-B wherein polymer block A represents a linear pharmaceutically acceptable hydrophilic polymer with a molecular weight <1,000, and polymer block B represents a polymer comprising at least two different monomers selected from glycolic acid, propiolactone, γ-butyrolactone, δ-valerolactone,

γ-valerolactone, ε-caprolactone, trimethylene carbonate, p-dioxanone, tetramethylene carbonate, ε-lactone, 1, 5-di-oxepan-2-one <u>characterized in that</u> the diblock copolymer is liquid at a temperature below 50°C.

A polymer can be considered as a molecule consisting of several (at least more than 2) repeating monomer units. Since block A as well as block B are polymers, they both consist of several monomer units linked to one another.

Polymer block B as described hereinabove may be composed of, among others, propiolactone, γ-butyrolactone, δ-valerolactone, γ-valerolactone, ε-caprolactone, trimethylene carbonate, p-dioxanone, tetramethylene carbonate, ε-lactone. Propiolactone corresponds to 2-oxetanone, γ-butyrolactone corresponds to dihydro-2(3H)-furanone, δ-valerolactone corresponds to tetrahydro-2*H*-pyran-2-one, γ-valerolactone corresponds to 5-methyldihydro-2(3*H*)-furanone, ε-caprolactone corresponds to 2-oxepanone, trimethylene carbonate corresponds to 1,3-dioxan-2-one, p-dioxanone corresponds to 1,4-dioxan-2-one, tetramethylene carbonate corresponds to 1,3-dioxepan-2-one, ε-lactone corresponds to 1,4-dioxepan-2-one.

Because the monomers of the hydrophobic polymer block B are linked to each other by ester bonds, the polymer block B is hydrolysable under physiological conditions and hence can be considered to be biodegradable. Depending on the monomer, polymer block B can be composed of only one monomer or can be composed of a mixture of at least two different monomers. When composed of two different monomers, the ratio may vary from 99:1 to 1:99, most preferred is a ratio of about 50:50.

The present copolymers are liquid at a temperature below 50°C.

A polymer is considered to be liquid below 50°C when its glass transition temperature is below or equal to 50°C. Preferred copolymers of the present invention ate liquid at the body temperature of the species to which they are administered, i.e. 37°C for human use. A polymer is considered to be liquid at 37°C when its glass transition temperature is below or equal to 37°C, preferably the glass transition temperature is below 37°C. More preferred copolymers of the present invention are liquid at room temperature (20-25°C). A polymer is considered to be liquid at room temperature when its glass transition temperature is below or equal to room temperature, preferably the glass transition temperature is below room temperature.

The present diblock copolymers can easily be mixed, e.g. upon gentle agitation, with aqueous media resulting in spontaneous micelle formation. When the polymers are administered orally, the mixing-propulsive forces exerted on them by the gastro-intestinal tract may be sufficient to result in *in situ* micelle formation.

Although the present copolymers are characterized by being liquid below 50°C. this does not exclude similar solid diblock copolymers from the ambit of the invention as long as they are self-emulsifying allowing to prepare micellar solutions or drug loaded micellar solutions without the need of surfactants, extensive heat, organic solvents, complex or time consuming processes.

Of particular interest is a diblock copolymer as described hereinabove wherein polymer block B represents a copolymer comprising at least two different monomers selected from glycolic acid, propiolactone, γ-butyrolactone, δ-valerolactone, γ-valerolactone, ε-caprolactone, trimethylene carbonate, p-dioxanone, tetramethylene carbonate, ε-lactone, 1, 5-dioxepan-2-one.

A further interesting embodiment is a diblock copolymer as described hereinabove wherein polymer block B represents a polymer comprising monomers of trimethylene carbonate and monomers selected from glycolic acid, propiolactone, γ-butyrolactone, δ-valerolactone, γ-valerolactone, ε-caprolactone, trimethylene carbonate, p-dioxanone, tetramethylene carbonate, ε-lactone, 1, 5-dioxepan-2-one or mixtures thereof.

Also an interesting embodiment is a diblock copolymer as described above wherein polymer block B represents a polymer comprising monomers selected from propiolactone, γ-butyrolactone, δ-valerolactone, γ-valerolactone, ε-caprolactone, trimethylene carbonate, p-dioxanone, tetramethylene carbonate, ε-lactone, 1,5-dioxepan-2-one or mixtures thereof.

A further interesting embodiment is a diblock copolymer as described above wherein polymer block B represents a copolymer comprising at least two different monomers selected from propiolactone, γ-butyrolactone, δ-valerolactone, γ-valerolactone, ε-caprolactone, trimethylene carbonate, p-dioxanone, tetramethylene carbonate, ε-lactone, 1, 5-dioxepan-2-one.

Yet a further interesting embodiment is a diblock copolymer as described above wherein polymer block B comprises two different monomers selected from propiolactone, γ-butyrolactone, δ-valerolactone, γ-valerolactone, ε-caprolactone, trimethylene carbonate, p-dioxanone, tetramethylene carbonate, ε-lactone, 1, 5-dioxepan-2-one.

Also an interesting embodiment is a diblock copolymer as described hereinabove wherein polymer block B is linear, in particular wherein polymer block B represents a copolymer comprising monomers selected from glycolic acid, propiolactone, γ-butyrolactone, δ-valerolactone, ε-caprolactone, trimethylene carbonate, p-dioxanone, tetramethylene carbonate, ε-lactone, 1, 5-dioxepan-2-one or mixtures thereof, more in particular wherein polymer block B represents a copolymer comprising at least two different monomers selected from glycolic acid, propiolactone, γ-butyrolactone, δ-valerolactone, ε-caprolactone, trimethylene carbonate, p-dioxanone, tetramethylene carbonate, ε-lactone, 1, 5-dioxepan-2-one. Also interesting are those diblock copolymers as described above wherein polymer block B represents a polymer comprising monomers selected from propiolactone, γ-butyrolactone, δ-valerotactone, ε-caprolactone, trimethylene carbonate, p-dioxanone, tetramethylene carbonate, ε-lactone, 1, 5-dioxepan-2-one or mixtures thereof or wherein polymer block B represents a polymer comprising monomers of trimethylene carbonate and monomers selected from glycolic acid, propiolactone, γ-butyrolactone, δ-valerolactone, ε-caprolactone, trimethylene carbonate, p-dioxanone, tetramethylene carbonate, ε-lactone, 1, 5-dioxepan-2-one or mixtures thereof or wherein polymer block B represents a copolymer comprising at least two different monomers selected from propiolactone, γ-butyrolactone, δ-valerolactone, ε-caprolactone, trimethylene carbonate, p-dioxanone, tetramethylene carbonate, ε-lactone, 1, 5-dioxepan-2-one or wherein polymer block B comprises two different monomers selected from propiolactone, γ-butyrolactone, δ-valerolactone, ε-caprolactone, trimethylene carbonate, p-dioxanone, tetramethylene carbonate, ε-lactone, 1, 5-dioxepan-2-one.

When the copolymers or the polymer blocks are described as being linear, this means that the copolymers or the polymer blocks consist of straight (not-branched) chains. The term "linear" polymer/copolymer is well known by a person skilled in the art.

A preferred embodiment is a diblock copolymer as described above wherein polymer block B comprises monomers selected from ε-caprolactone and trimethylene carbonate, in particular in a ratio of about 50:50.

An interesting embodiment of the hydrophilic polymer block A is poly($C_{1-20}$alkylene oxide) or a derivative thereof.

As used hereinabove or hereinafter $C_{1-20}$alkylene as a group or part of a group defines straight chain saturated bivalent hydrocarbon radicals having from 1 to 20 carbon atoms such as methylene, 1,2-ethanediyl or 1,2-ethylidene, 1,3-propanediyl or 1,3-propylidene, 1,4-butanediyl or 1,4-butylidene, 1,5-pentylidene, 1,6-hexylidene, 1,7-heptylidene, 1,8-octylidene, 1,9-nonylidene, 1-10-decylidene and the like. Thus, poly($C_{1-20}$alkylene oxide) encompasses for instance poly(ethylene oxide) or poly(ethylene glycol) or poly(propylene oxide) or mixtures thereof. Poly(ethylene oxide) or poly (ethylene glycol) may be used interchangeably and shall mean a polymer of ethylene glycol or hydrated ethylene oxide. Whenever appropriate $C_{1-20}$alkylene may additionally also define branched chain saturated bivalent hydrocarbon radicals having from 1 to 20 carbon atoms such as 1,3-2-methyl-propanediyl; 1,6-2-methyl-3-methyl-hexylidene and the like.

As used hereinabove or hereinafter $C_{1-4}$alkyl as a group or part of a group defines straight chain saturated monovalent hydrocarbon radicals having from 1 to 4 carbon atoms such as methyl, ethyl, propyl, butyl; $C_{1-10}$alkyl as a group or part of a group defines straight chain saturated hydrocarbon radicals having from 1 to 10 carbon atoms such as the group defined for $C_{1-4}$alkyl and pentyl, hexyl, pentyl, hexyl, heptyl, octyl, nonyl, decyl; $C_{1-20}$alkyl as a group or part of a group defines straight chain saturated monovalent hydrocarbon radicals having from 1 to 20 carbon atoms such as the group defined for $C_{1-10}$alkyl and undecyl, dodecyl and the like. Whenever appropriate $C_{1-4}$alkyl, $C_{1-10}$alkyl and $C_{1-20}$ alkyl may additionally also define branched chain saturated monovalent hydrocarbon radicals such as 2-methylpropyl; 2-methyl-3-methylbutyl and the like.

As used hereinabove or hereinafter, a derivative of poly($C_{1-20}$alkylene oxide) means an end-capped poly ($C_{1-20}$alkylene oxide) wherein the reactive group at one side of the polymer is protected by way of a suitable protective group, for instance a $C_{1-20}$alkyl group (e.g. methyl, octyl, nonyl, decyl, dodecyl) or benzyl. A trialkylsilyl group (e.g. *tert*-butyldimethylsilyl, *tert*-butyldiphenylsilyl or trimethylsilyl) or trityl or tetrahydropyranyl or p-nonylphenyl or [4-(1,1,3,3-tetramethylbutyl)phenyl], also belong to the ambit of the invention. The reactive group at the other side of the polymer is left unprotected and hence capable of further reaction. An example of a poly(ethylene glycol) or an end-capped derivative thereof is a poly(ethylene glycol) of formula $R^1$-($OCH_2CH_2$)$_n$-OH wherein $R^1$ is hydrogen or $C_{1-20}$alkyl or benzyl. $R^1$ may also represent a trialkylsilyl group or trityl or tetrahydropyranyl or p-nonylphenyl or [4-(1,1,3,3-tetramethylbutyl)phenyl], and n is an integer larger than 2.

The use of protecting groups is fully described in 'Protective Groups in Organic Chemistry', edited by J W F McOmie, Plenum Press (1973), and 'Protective Groups in Organic Synthesis' 2nd edition, T W Greene & P G M Wutz, Wiley Interscience (1991).

An interesting embodiment of the present invention is a diblock copolymer of formula A-B as described above wherein polymer block A is poly(ethylene glycol) or a derivative thereof, more in particular a poly(ethylene glycol) of formula $R^1$-($OCH_2CH_2$)$_n$-OH wherein $R^1$ is hydrogen or $C_{1-20}$alkyl, in particular $C_{1-20}$alkyl, more in particular $C_{1-10}$alkyl, even more in particular $C_{1-4}$alkyl and most in particular methyl; and n is an integer larger than 2, preferably from 8 to 100, more preferably from 8 to 50, most preferred from 8 to 20. The most preferred hydrophilic polymer is poly(ethylene glycol) monomethylether.

Poly(ethylene glycol) or a derivative thereof was chosen as a preferred hydrophilic polymer block A because of its biocompatibility and its non-toxicity and rapid clearance from the body.

Within the ambit of the invention, the hydrophilic polymer block A may also represent polyvinyl alcohol; polyvinyl pyrrolidone; polyacrylamide, polymethacrylamide, poly(*N*-(2-hydroxypropyl)methacrylamide, poly(*N*-isopropylacrylamide) or analogues of these polymers; dextran; gelatine; alginic acid; sodium alginate or derivatives of these hydrophilic polymers or copolymers of two or more of the monomers from which these hydrophilic polymers are derived. A derivative of a hydrophilic polymer as described above shall, in case of a hydrophilic polymer having two reactive groups, mean an end-capped hydrophilic polymer wherein the reactive group at one side of the polymer is protected by way of a suitable leaving group, leaving the reactive group at the other side of the polymer unprotected and hence capable of further reaction. When several reactive groups are present in the hydrophilic polymer, a derivative of the hydrophilic polymer shall mean a protected hydrophilic polymer wherein at least one reactive group is unprotected in order to ensure further reactivity of the hydrophilic polymer. Reactive groups which it is desirable to protect include in addition to what is mentioned hereinabove, amino and carboxylic acid. Suitable protecting groups for amino include tert-butyloxycarbonyl or benzyloxycarbonyl. Suitable protecting groups for carboxylic acid include $C_{1-4}$alkyl or benzyl esters.

The self-emulsifying properties of the present diblock copolymers are more pronounced when there is a good balance between the hydrophilic and the hydrophobic part of the diblock copolymer in order to make the polymers more readily mixable with an aqueous medium.

Of particular interest is a hydrophilic polymer block A with a molecular weight < 1,000, most preferred ranging from > 350 to ≤ 750, more in particular poly(ethylene glycol) or a derivative thereof with a molecular weight ≤ 1,000, even more in particular poly(ethylene glycol) or a derivative thereof with a molecular weight ranging from > 350 to ≤ 750, most in particular poly(ethylene glycol) or a derivative thereof with a molecular weight of 750. Preferred is poly(ethylene glycol) methylether (also indicated as poly(ethylene glycol) monomethylether) with a molecular weight of 550 or 750. Most preferred is poly(ethylene glycol) monomethylether with a molecular weight of 750.

Since the polymers of the present invention are characterized by being liquid below 50°C, polymers with a limited molecular weight are preferred. Of particular interest are those diblock copolymers of formula A-B having a molecular weight ranging from 2,000 to 100,000, in particular a molecular weight ranging from 2,000 to 75,000, more in particular a molecular weight ranging from 2,000 to 50,000, even more in particular a molecular weight ranging from 2,000 to 25,000, further in particular a molecular weight ranging from 2,000 to 20,000, yet further in particular a molecular weight ranging from 2,000 to 15,000, preferred a molecular weight ranging from 2,000 to 10,000, more preferred a molecular weight ranging from 2,000 to 8,000, even more preferred a molecular weight ranging from 2,500 to 7,000.

Another aspect of the invention relates to a composition comprising an active ingredient and one or more diblock copolymers of formula A-B wherein polymer block A represents a pharmaceutically acceptable hydrophilic polymer and polymer block B represents a polymer comprising monomers selected from glycolic acid, propiolactone, γ-butyrolactone,

δ-valerolactone, γ-valerolactone, ε-caprolactone, trimethylene carbonate, p-dioxanone, tetramethylene carbonate, ε-lactone, 1, 5-dioxepan-2-one or mixtures thereof

characterized in that the diblock copolymer is liquid below 50°C and the composition is liquid below 50°C. In particular, the composition is non-aqueous, meaning that it does not contain substantial amounts of water or an aqueous solution. Thus, in general the compostion will preferably be substantially water-free, e.g. containing up to 3% by weight water, preferably less than 1 % by weight water, and most preferably less than 0.5% water. Preferably, the active ingredient is not covalently bound to the one or more diblock copolymers.

More in particular, the present invention relates to a composition comprising a pharmaceutically active ingredient and any one of the diblock copolymers of formula A-B as described hereinabove. Preferably, the composition is liquid at room temperature or at 37°C.

A "liquid" composition is well-known to the person skilled in the art.

The present compositions may comprise as outlined above one or more diblock copolymers of formula A-B. These diblock copolymers may be linear or branched. Thus, a composition comprising a mixture of linear and branched diblock copolymers are also encompassed by the present invention. Preferably, the diblock copolymers present in the present compositions are linear.

To prepare the compositions of the invention the active ingredient and the one or more diblock copolymers of formula A-B are intimately admixed, for instance by simple stirring. Preferably, the active ingredient is dissolved in the liquid diblock copolymer.

Although the present compositions are characterized by being liquid below 50°C, this does not exclude similar solid compositions from the ambit of the invention as long as they are self-emulsifying allowing to prepare drug loaded micellar solutions without the need of surfactants, extensive heat, organic solvent, complex or time consuming processes.

The term active ingredient comprises a drug or a pharmaceutically active ingredient or a cosmetic active ingredient.

Examples of active ingredients are:

- analgesic and anti-inflammatory drugs (NSAIDs, fentanyl, indomethacin, ibuprofen, ketoprofen, nabumetone, paracetamol, piroxicam, tramadol, COX-2 inhibitors such as celecoxib and rofecoxib) ;
- anti-arrhythmic drugs (procainamide, quinidine, verapamil) ;
- antibacterial and antiprotozoal agents (amoxicillin, ampicillin, benzathine penicillin, benzylpenicillin, cefaclor, cefadroxil, cefprozil, cefuroxime axetil, cephalexin, chloramphenicol, chloroquine, ciprofloxacin, clarithromycin, clavulanic acid, clindamycin, doxyxycline, erythromycin, flucloxacillin sodium, halofantrine, isoniazid, kanamycin sulphate, lincomycin, mefloquine, minocycline, nafcillin sodium, nalidixic acid, neomycin, norfloxacin, ofloxacin, oxacillin, phenoxymethylpenicillin potassium, pyrimethamine-sulfadoxime, streptomycin, *N*-[[(5S)-3-[4-(2,6-dihydro-2-methylpyrrolo[3,4-c]pyrazol-5(4*H*)-yl)-3-fluorophenyl]-2-oxo-5-oxazolidinyl]methyl]-acetamide (CA Index name :

474016-05-2);

- anti-coagulants (warfarin) ;
- antidepressants (amitriptyline, amoxapine, butriptyline, clomipramine, desipramine, dothiepin, doxepin, fluoxetine, reboxetine, amineptine, selegiline, gepirone, imipramine, lithium carbonate, mianserin, milnacipran, nortriptyline, paroxetine, sertraline ; 3-[2-[3,4-dihydrobenzofuro[3,2-c]pyridin-2(1*H*)-yl]ethyl]-2-methyl-4*H*-pyrido[1,2-a]pyrimidin-4-one;   3-[[4-[(2E)-3-(4-fluorophenyl)-2-methyl-2-propenyl]-1-piperazinyl]methyl]-3a,4-dihydro-7,8-dimethoxy-3H-[1]Benzopyrano[4,3-c]-isoxazole [(3R,3aS)-rel-(+)] (CA Index name : 452314-01-1);
- anti-diabetic drugs (glibenclamide, metformin, (Z) 5-[[3-(5,6,7,8-tetrahydro-3,5,5,8,8-pentamethyl-2-naphthalenyl)-4-(trifluoromethoxy)phenyl]methylene]-2,4-thiazolidinedione (CA Index name : 329215-18-1);
- anti-epileptic drugs (carbamazepine, clonazepam, ethosuximide, gabapentin, lamotrigine, levetiracetam, phenobarbitone, phenytoin, primidone, tiagabine, topiramate, valpromide, vigabatrin) ;
- antifungal agents (amphotericin, clotrimazole, econazole, fluconazole, flucytosine, griseofulvin, itraconazole, ketoconazole, miconazole nitrate, nystatin, terbinafine, voriconazole) ;
- antihistamines (astemizole, cinnarizine, cyproheptadine, decarboethoxyloratadine, fexofenadine, flunarizine, levocabastine, loratadine, norastemizole, oxatomide, promethazine, terfenadine) ;
- anti-hypertensive drugs (captopril, enalapril, ketanserin, lisinopril, minoxidil, prazosin, ramipril, reserpine, terazosin) ;
- anti-muscarinic agents (atropine sulphate, hyoscine);
- antineoplastic agents and antimetabolites (platinum coordination compounds such as cisplatin, carboplatin or oxalyplatin; taxane compounds such as paclitaxel or docetaxel; topoisomerase I inhibitors such as camptothecin compounds for example irinotecan or topotecan; topoisomerase II inhibitors such as anti-tumour podophyllotoxin derivatives for example etoposide or teniposide; anti-tumour vinca alkaloids such as vinblastine, vincristine or vinorelbine; anti-tumour nucleoside derivatives such as 5-fluorouracil, gemcitabine or capecitabine; alkylating agents such as nitrogen mustard or nitrosourea for example cyclophosphamide, chlorambucil, carmustine or lomustine; anti-tumour anthracycline derivatives such as daunorubicin, doxorubicin, idarubicin or mitoxantrone; HER2 antibodies such as trastuzumab; estrogen receptor antagonists or selective estrogen receptor modulators such as tamoxifen, toremifene, droloxifene, faslodex or raloxifene; aromatase inhibitors such as exemestane, anastrozole, letrazole or vorozole; differentiating agents such as retinoids, vitamin D and retinoic acid metabolism blocking agents (RAMBA) for example accutane; DNA methyl transferase inhibitors such as azacytidine; kinase inhibitors for example flavopetidol, imatinib mesylate, gefitinib or N3-[4-(aminosulfonyl)phenyl]-1-(2,6-difluorobenzoyl)- 1*H*-1,2,4-triazole-3,5-diamine (CA Index name : 443797-96-4); famesyltransferase inhibitors; HDAC inhibitors such as short-chain fatty acids for example butyrate, 4-phenylbutyrate or valproic acid or hydroxamic acids for example suberoylanilide hydroxamic acid (SAHA), biaryl hydroxamate A-161906, bicyclic aryl-*N*-hydroxycarboxamides, pyroxamide, CG-1521, PXD-101, sulfonamide hydroxamic acid, LAQ-824, trichostatin A (TSA), oxamflatin, scriptaid, m-carboxy cinnamic acid bishydroxamic acid, or trapoxin-hydroxamic acid analogue or cyclic tetrapeptides for example trapoxin, apidicin or depsipeptide or benzamides for example MS-275 or CI-994, or depudecin);
- anti-migraine drugs (alniditan, naratriptan, sumatriptan) ;
- anti-Parkinsonian drugs (bromocryptine mesylate, levodopa, selegiline) ;
- antipsychotic, hypnotic and sedating agents (alprazolam, buspirone, chlordiazepoxide, chlorpromazine, clozapine, diazepam, flupenthixol, fluphenazine, flurazepam, 9-hydroxyrisperidone, lorazepam, mazapertine, olanzapine, oxazepam, pimozide, pipamperone, piracetam, promazine, risperidone, selfotel, seroquel, sertindole, sulpiride, temazepam, thiothixene, triazolam, trifluperidol, ziprasidone, zolpidem) ;
- anti-stroke agents (lubeluzole, lubeluzole oxide, riluzole, aptiganel, eliprodil, remacemide) ;
- antitussive (dextromethorphan, laevodropropizine) ;
- antivirals (acyclovir, ganciclovir, loviride, tivirapine, zidovudine, lamivudine, zidovudine + lamivudine, zidovudine+lamivudine+abacavir, didanosine, zalcitabine, stavudine, abacavir, lopinavir, lopinavir+ritonavir, amprenavir, nevirapine, efavirenz, delavirdine, indinavir, nelfinavir, ritonavir, saquinavir, adefovir, hydroxyurea, TMC 125, TMC 120, 4-[[4-[4-(2-cyanoethenyl)-2,6-dimethylphenyl]amino]-2-pyrimidinyl]amino]benzonitrile);
- beta-adrenoceptor blocking agents (atenolol, carvedilol, metoprolol, nebivolol, propanolol) ;
- cardiac inotropic agents (amrinone, digitoxin, digoxin, milrinone) ;
- corticosteroids (beclomethasone dipropionate, betamethasone, budesonide, dexamethasone, hydrocortisone, methylprednisolone, prednisolone, prednisone, triamcinolone) ;
- disinfectants (chlorhexidine) ;
- diuretics (acetazolamide, frusemide, hydrochlorothiazide, isosorbide);
- enzymes ;
- essential oils (anethole, anise oil, caraway, cardamom, cassia oil, cineole, cinnamon oil, clove oil, coriander oil, dementholised mint oil, dill oil, eucalyptus oil, eugenol, ginger, lemon oil, mustard oil, neroli oil, nutmeg oil, orange oil, peppermint, sage, spearmint, terpineol, thyme) ;
- gastro-intestinal agents (cimetidine, cisapride, clebopride, diphenoxylate, domperidone, famotidine, lansoprazole, loperamide, loperamide oxide, mesalazine, metoclopramide, mosapride, nizatidine, norcisapride, olsalazine, omeprazole, pantoprazole, perprazole, prucalopride, rabeprazole, ranitidine, ridogrel, sulphasalazine) ;
- haemostatics (aminocaproic acid) ;
- lipid regulating agents (atorvastatin, lovastatin, pravastatin, probucol, simvastatin) ;
- local anaesthetics (benzocaine, lignocaine) ;
- opioid analgesics (buprenorphine, codeine, dextromoramide, dihydrocodeine, hydrocodone, oxycodone, morphine);
- parasympathomimetics and anti-dementia drugs (AIT-082, eptastigmine, galanthamine, metrifonate, milameline, neostigmine, physostigmine, tacrine, donepezil, rivastigmine, sabcomeline, talsaclidine, xanomeline, memantine, lazabemide) ;
- peptides and proteins (antibodies, becaplermin, cyclosporine, erythropoietin, immunoglobulins, insuline);
- sex hormones (oestrogens : conjugated oestrogens, ethinyloestradiol, mestranol, oestradiol, oestriol, oestrone ; progestogens ; chlormadinone acetate, cyproterone acetate, 17-deacetyl norgestimate, desogestrel, dienogest, dydrogesterone, ethynodiol diacetate, gestodene, 3-keto desogestrel, levonorgestrel, lynestrenol, medroxy-progesterone acetate, megestrol, norethindrone, norethindrone acetate, norethisterone, norethisterone acetate, norethynodrel, norgestimate, norgestrel, norgestrienone, progesterone, quingestanol acetate) ;
- stimulating agents, phosphodiesterase 5 inhibitors (sildenafil; 3-(2,3-dihydro-5-benzofuranyl)-1,2,3,4-tetrahydro-2-(2-pyridinyl)-9*H*-pyrrolo[3,4-b]quinolin-9-one, (3R) (CA Index name : 374927-41-0) or its mono methanesulfonate salt; 3-(2,3-dihydro-5-benzofuranyl)-1,2,3,4-tetrahydro-2-[5-(2-pyridinyl)-2-pyrimidinyl]-9*H*-pyrrolo[3,4-b]quinolin-9-one, (3R) (CA Index name : 374927-06-7)
- vasodilators (amlodipine, buflomedil, amyl nitrite, diltiazem, dipyridamole, glyceryl trinitrate, isosorbide dinitrate, lidoflazine, molsidomine, nicardipine, nifedipine, oxpentifylline, pentaerythritol tetranitrate);

their N-oxides, their pharmaceutically acceptable acid or base addition salts and their stereochemically isomeric forms.

Preferably, the active ingredient used in the compositions of the invention may be any organic or inorganic material which is no more than sparingly soluble, i.e. which is sparingly soluble, slightly soluble, very slightly soluble, or practically insoluble in pure water at 21°C (ie. requiring from 30, from 100, from 1000 or from 10000 parts water to put 1 part by weight of the active drug compound into solution).

The compositions of the present invention may be formulated into various pharmaceutical dosage forms for administration purposes.

Hence, the present invention also relates to a pharmaceutical dosage form comprising a therapeutically effective amount of a composition according to the invention.

For instance, the present compositions can be filled as such in a suitable capsule, such as for example a gelatine capsule. When orally administered, the capsule dissolves in the gastro-intestinal fluids and the composition comprising the active ingredient and the diblock copolymer forms a drug loaded micellar solution upon contact with the aqueous gastro-intestinal fluids and upon mild agitation in the gastro-intestinal tract. The present compositions may also be filled into a suitable container, such as for example a vial. Just before administration, for instance via the parenteral route or via the oral route, the composition may be diluted with a suitable diluent and subsequently administered.

As further appropriate dosage forms there may be cited all compositions usually employed for systemically or topically administering drugs.

To prepare the pharmaceutical dosage forms of this invention, an effective amount of the composition of the present invention is formulated into a pharmaceutical dosage form. For instance, the compositons may be combined in intimate admixture with a pharmaceutically acceptable carrier, which carrier may take a wide variety of forms depending on the form of preparation desired for administration.

The pharmaceutical dosage forms are desirable in unitary dosage form suitable, particularly, for administration orally, rectally, percutaneously, or by parenteral injection. For example, in preparing the compositions in oral dosage form, any of the usual pharmaceutical media may be employed such as, for example water, in the case of oral liquid preparations; or solid carriers such as starches, sugars, kaolin, diluents, lubricants, binders, disintegrating agents and the like in the case of powders, pills, capsules, and tablets. Capsules or oral solutions represent the most advantageous oral unit dosage forms. As already indicated above, the present compositions may also be filled as such into capsules.

For parenteral compositions, the carrier will usually comprise sterile water, at least in large part, though other ingredients may be included. Injectable solutions, for example, may be prepared in which the carrier comprises saline solution, glucose solution or a mixture of saline and glucose solution.

Also included, as already indicated above, are solid or liquid preparations which are intended to be converted, shortly before use, to liquid or diluted liquid form preparations.

In the compositions suitable for percutaneous, transdermal administration, the carrier optionally comprises a penetration enhancing agent and/or a suitable wetting agent, optionally combined with suitable additives of any nature in minor proportions, which additives do not introduce a significant deleterious effect on the skin. Said additives may facilitate the administration to the skin and/or may be helpful for preparing the desired compositions.

The compositions or dosage forms of the present invention may also be administered via inhalation or insufflation by means of methods and formulations employed in the art for administration via this way. Thus, in general the compositions or dosage forms of the present invention may be administered to the lungs in the form of a solution. Any system developed for the delivery of solutions or dry powders via oral or nasal inhalation or insufflation are suitable for the administration of the present compounds.

The dosage forms of the present invention can also be used as a diagnostic dosage form. The active ingredient of the present compositions can be a labeled ingredient which can, upon administration, interact with or which is taken up by a specific target tissue or organ. It thereby enhances radiographic, magnetic resonance and ultrasound imaging at the target tissue or organ.

Depending on the active ingredient, the present dosage forms can also be applied as cosmetic preparations, for instance when anti-aging, anti-wrinkling agents or antoxidantia are included. They can also be used as a sunscreen.

Preferably, the dosage forms of the present invention are suitable for oral, parenteral or transdermal administration, most preferably oral or parenteral administration.

Preferably, the dosage form is an aqueous solution. Because the copolymers of the present invention are self-emulsifying, said aqueous solution can be prepared at relatively low temperature, i.e. below 50°C, without the need of organic solvents, complex or time consuming manufacturing processes. Therefore, the present invention also relates to a process to prepare an aqueous solution comprising an active ingredient and one or more diblock copolymers of formula A-B as described hereinabove characterized by mixing the active ingredient with the one or more liquid copolymers, i.e. at a temperature below 50°C, followed by addition of water while stirring. Preferably, the stirring time is limited to at most 24 hours. Also preferred is the mixing of the active ingredient with the one or more liquid copolymers at a temperature up to 37 °C, most preferred is mixing at room temperature.

The present invention also relates to a process to prepare an aqueous solution comprising an active ingredient and one or more diblock copolymers of formula A-B as described hereinabove characterized by

    a) mixing the one or more copolymers with water at a temperature below 50°C, followed by
    b) the addition of the active ingredient to the aqueous polymeric solution obtained under a) while stirring.

Preferably, the stirring time is limited to at most 24 hours. Also preferred is mixing the one or more copolymers with water at a temperature up to 37 °C, most preferred is mixing at room temperature.

The exact dosage and frequency of administration depends on the particular active ingredient used, the desired dissolution profile, the particular condition being treated, the severity of the condition being treated, the age, weight and general physical condition of the particular patient as well as other medication the individual may be taking, as is well known to those skilled in the art. Furthermore, it is evident that the effective daily amount may be lowered or increased depending on the response of the treated subject and/or depending on the evaluation of the physician prescribing the dosage forms of the instant invention.

Viewed from a further aspect the invention also provides the use of a composition according to the present invention for the manufacture of a pharmaceutical dosage form for administration, in particular for oral or parenteral administration, to a human or non-human animal in need of treatment.

It also relates to the use of a composition of the invention for the manufacture of a pharmaceutical dosage form, in particular for the manufacture of a pharmaceutical dosage form for use in a method of therapy or diagnosis of the human or non-human animal (e.g. mammalian, reptilian or avian) body, in particular for oral or parenteral administration to a human or non-human animal in need of treatment.

Viewed from a still further aspect the invention provides a method of therapy or diagnosis of the human or non-human animal (e.g. mammalian, reptilian or avian) body which comprises administering to said body a therapeutically or diagnostically effective dose of a composition according to the present invention.

This invention also relates to a pharmaceutical package suitable for commercial sale comprising a container, a pharmaceutical dosage form as described above, and associated with said package written matter.

The invention will now be described further in detail in the following non-limiting

Examples.

Preparation of the diblock copolymers.

The diblock copolymers of the present invention were synthesized by a ring opening polymerization process in the presence of a suitable catalyst according to the method described in US 5,653,992 and US 5,631,015 (Bezwada et al.). Typical catalysts include stannous octoate, antimony oxide, tin chloride, tin(II) 2-ethylhexanoate, dibutyltin oxide, aluminium isopropoxide, yttrium isopropoxide, sodium, potassium, potassium t-butoxide, sodium t-butoxide and the like. Preferred catalyst is stannous octoate. The reaction is performed at elevated temperature ranging from 80°C to 180 °C and the reaction time may vary between several hours to several days, preferable between 8 and 24 hours.

*Preparation of diblock copolymer D1.1 (see Table 1)*

In the reaction flask, 7.6 μmol of stannous octoate solution in toluene (0.33M), 187.5 mmol trimethylene carbonate (monomer), 187.5 mmol ε-caprolactone (monomer) and PEG-550 monomethylether (mmePEG550) (initiator) in a molar ratio monomer to initiator of 13 to 1 were added and heated to 160°C for 24 hours. After completion of the reaction, the polymer was heated under vacuum to remove unreacted monomer.

Characterization of the diblock copolymers

The polymer composition and residual monomer content were analyzed by proton NMR. Therefore, the copolymers were dissolved in hexafluoroacetone sesquideuterate and deuterobenzene or deuterated chloroform. Subsequently spectra were taken employing a Unity-Plus 400 NMR spectrometer. The ratio of the various monomers in the polymer were determined by integrating the methylene and methyl resonance's in the 0 to 7.5 ppm spectral region and calculating the mole percent of each monomer in the polymer from the normalized surface area of the respective monomers (polymerized and monomer form).
Gel permeation chromatography (GPC) was employed to determine the molecular weight and the polydispersity of the polymers. A Waters Alliance 2690 separation module equipped with a Wyatt Optilab DSP refractometer, a Dawn multi-angle laser photometer (Wyatt), and Waters Styragel HR 3-4 columns was used. Polystyrene standards were used for calibration. HPLC grade tetrahydrofuran or hexafluoroisopropanol were used as solvent and mobile phase.
Table 1 lists diblock copolymers prepared according to the method described above. The results indicate that the synthesis has a good reproducibility (see for instance D4.1, D4.3 and D4.4).

**Table 1**: Physicochemical characteristics of the diblock copolymers (All the copolymers were liquid below 50°C).

| Name | Monomer ratio at the start of polymerization (mol/mol) | Monomer ratio in final polymer (mol/mol) | Reaction time (h) | Initiator | Molar ratio Monomer/ Initiator | Mw | PD |
|---|---|---|---|---|---|---|---|
| D 1.1 | CAP/TMC 50/50 | 50.1 % CAP 49.6%TMC | 24 | mmePEG 550 | 13 to 1 | 4641 | 2.1 |
| D 1.2 | CAP/TMC 50/50 | 49.3% CAP, 50.6% TMC | 24 | mmePEG 550 | 13 to 1 | 5504 | 1.92 |
| D 1.3 | CAP/TMC 50/50 | 49.3 % CAP, 50.4 %TMC | 24 | mmePEG 550 | 13 to 1 | 5507 | 1.43 |
| D 1.4 | CAP/TMC 50/50 | 48.9% CAP, 50.3% TMC | 16 | mmePEG 550 | 13 to 1 | 5045 | 1.77 |
| D1.5 | CAP/TMC 50/50 | 49.0 % CAP, 50.3 %TMC | 8 | mmePEG 550 | 13 to 1 | 4926 | 1.90 |
| D1.6 | CAP/TMC 50/50 | 49.1% CAP, 50.8% TMC | 8 | mmePEG 550 | 13 to 1 | 5046 | 1.79 |
| D 2 | CAP/TMC 50/50 | 48.5% CAP, 51.3% TMC | 24 | mmePEG 550 | 8 to 1 | 3285 | 1.82 |
| D 4.1 | CAP/TMC 50/50 | 49.8% CAP, 50.0% TMC | 24 | mmePEG 750 | 13.3 to 1 | 6162 | 1.97 |
| D 4.2 | CAP/TMC 50/50 | 48.9% CAP, 50.7% TMC | 8 | mmePEG 750 | 13.3 to 1 | 5274 | 1.79 |
| D 4.3 | CAP/TMC 50/50 | 49.3% CAP, 49.1 % TMC | 24 | mmePEG 750 | 13.3 to 1 | 4815 | 1.91 |
| D 4.4 | CAP/TMC 50/50 | 49.1% CAP, 50.7% TMC | 24 | mmePEG 750 | 13.3 to 1 | 5249 | 1.76 |

(continued)

| Name | Monomer ratio at the start of polymerization (mol/mol) | Monomer ratio in final polymer (mol/mol) | Reaction time (h) | Initiator | Molar ratio Monomer/ Initiator | Mw | PD |
|---|---|---|---|---|---|---|---|
| D 5 | CAP/TMC 50/50 | 49.0% CAP, 50.8% TMC | 8 | mmePEG 750/ mmePEG 550 (1/3) | 13.3 to 1 | 5075 | 1.80 |
| D 6 | CAP/TMC 50/50 | 48.6% CAP, 51% TMC | 24 | mmePEG 2000 | 13.3 to 1 | 6500 | 1.9 |

D = diblock copolymer; CAP = ε-caprolactone; TMC = trimethylene carbonate; PEG = Poly(ethylene glycol); mmePEG = Poly(ethylene glycol) monomethylether ; PD=Polydispersity; $M_w$ = weight average molecular weight; PD and $M_w$ were determined by GPC. Monomer ratios in the final polymer were determined by $^1$H-NMR.

Characterization of aqueous micellar solutions of the diblock copolymers

Since the diblock copolymers are intended to be used for their self-emulsifying properties, i.e. their ability to spontaneously form micelles in water, aqueous solutions of the diblock copolymers were examined. The size and shape of the micelles, and the critical micellar concentration, i.e. the concentration from which on micelles are formed, were determined.

*Size of the micelles*

The size of the micelles of 100 mg/ml diblock copolymer solutions in water was determined by photon correlation spectroscopy using a Coulter N4MD or Malvern autosizer 4700 at 25°C.

The results are gathered in Table 2. The size of the self-aggregated structures formed by the addition of water to the diblock copolymers was in the range of 15 to 125 nm. Micelles made of copolymers having the same starting composition but which were coming from different batches (copolymers D4.1, D4.3 and D4.4, see Table 1), were found to have very similar size (varying between 15 and 23 nm).

In order to determine the shape of the micelles, cryo transmission electron microscopy (cryo-TEM) observations were also made (copolymers D2 and D4.3). Therefore, 10 mg/ml polymer aqueous solution were prepared. A small droplet of the solution was placed on a TEM-grid. The excess of solution was eliminated with a filter paper in order to obtain a thin film (< 100 nm). The sample was then plunged rapidly in cryogenic liquid ethane. The grid was transferred and mounted under liquid nitrogen on a cryo-TEM holder that was inserted into a TEM Philips CM12. The analysis were performed at -172°C, at 120 kV. The pictures obtained in this way clearly revealed spherical structures.

*Critical micellar concentration (CMC) determination*

In order to investigate the CMC of the diblock copolymers, the surface tension of aqueous solutions containing increasing amounts of diblock copolymer ($10^{-8}$ to $10^{-3}$ g/ml) was measured by the ring method (Du Noüy tensiometer) at 37°C. The surface tension of the solutions decreased with increasing polymer concentration until the surface tension remained constant, indicating that a CMC was reached. The CMC was determined at the intersection of 2 linear regression lines.

The results are gathered in Table 2. From these results it can be concluded that the CMC is sufficiently low to ensure that, if for instance 100 mg of polymer is administered orally, the concentration in the gastro-intestinal tract will remain well above the CMC of the copolymer, so that the polymer will remain in a micellar form in the gastro-intestinal tract, a prerequisite for increasing the solubility of the co-administered poorly water soluble drug.

*Stability of the micelles*

In order to evaluate the behavior of the micelles of the present diblock copolymers in gastric fluid, aqueous solutions of copolymers D4.1 and D4.2 containing increasing amounts of the copolymers were prepared at 37°C at pH 2 (the pH was adjusted with a 0.1M HCl solution) and the CMC was determined.

The CMC at pH 2 and 37°C was $5.10^{-5}$ g/ml for D4.1 and $4.1.10^{-5}$ g/ml for D4.2. These data confirm that the copolymers can form micelles under physiological conditions.

The influence of ionic strength on micelle formation by polymers D1.6, D4.2 and D5 was also assessed. In solutions of 0.09%, 0.9% and 9% (w/v) NaCl, the polymers were able to form micelles. Micelle formation in a 1% solution of albumin, and in FESSIF (fed state simulated intestinal fluid) buffer and FASSIF (fasted state simulated intestinal fluid) buffer was also determined. It was found that also in these media the polymers were able to form micelles. FESSIF and FASSIF buffer are well-known to the skilled person.

Micellization energy

The diblock copolymers used in the compositions of the present invention are characterized by having self-emulsifying properties. In order to support this statement, the micellization energy was determined from the critical micellar concentration by the following calculation :

$$\Delta G_0 = RT \ln X_{cmc}$$

With R = gas constant = 8.3143 J/K. mole
T = temperature in °K
$X_{cmc}$= concentration at CMC in molar fraction
$\Delta G_0$= micellization energy in kJ/mole

The calculated micellization energy values for different copolymers are reported in Table 2.

The negative values of the micellization energy indicate that the micellization is spontaneous (it did not require supplementary energy).

**Table 2:** Physicochemical characterization of aqueous solutions of the diblock copolymers

| Polymer | Size (nm) | CMC (ring method) (g/ml) | Micellisation energy (kJ/mol) |
|---|---|---|---|
| D1.2 | 125 | $2.9.10^{-5}$ | -41.6 |
| D1.3 | 90 | $9.3.10^{-5}$ | -38.8 |
| D1.4 | 94 | $10^{-4}$ | -38.2 |
| D1.5 | 92 | $5.10^{-4}$ | -34 |
| D1.6 | 76 | $1.7.10^{-5}$ | -43 |
| D2 | 31 | $6.3.10^{-5}$ | -37.7 |
| D4.1 | 20 | $1.2. 10^{-5}$ | -42.9 |
| D4.2 | 20 | $1.3.10^{-5}$ | -44.3 |
| D4.3 | 15 | $8.3.10^{-4}$ | -32.7 |
| D4.4 | 23 | $1.10^{-5}$ | -44.3 |
| D5 | 25 | $2.6.10^{-5}$ | -41.7 |
| D6 | 85 | $5.10^{-5}$ | -44.3 |

Solubility studies of poorly water soluble drugs in aqueous solutions of the diblock copolymers

The compositions of the present invention are able to form drug loaded micellar solutions upon addition to an aqueous medium. The drug loading capacity, the solubilization ability of the compositions of the present invention were determined.

The solubility of BCS (BioClassification System) class II model compounds in the micellar solutions was assessed: risperidone, ketoconazole, hydrocortisone, indomethacin, cyclosporin, amphotericin B, TMC 120, TMC 125, 4-[[4-[[4-(2-cyanoethenyl)-2,6-dimethylphenyl]amino]-2-pyrimidinyl]amino]benzonitrile, 3-[[4-[(2E)-3-(4-fluorophenyl)-2-methyl-2-propenyl]-1-piperazinyl]methyl]-3a,4-dihydro-7,8-dimethoxy-3*H*-[1]benzopyrano[4,3-c]isoxazole [(3R, 3aS)-rel-(+)] (CA Index name : 452314-01-1), *N*-[[(5S)-3-[4-(2,6-dihydro-2-methylpyrrolo[3,4-c]pyrazol-5(4*H*-yl)-3-fluorophenyl]-2-oxo-5-oxazolidinyl]methyl]-acetamide (CA Index name : 474016-05-2), (Z) 5-[[3-(5,6,7,8-tetrahydro-3,5,5,8,8-pentamethyl-2-naphthalenyl)-4-(trifluoromethoxy)phenyl]methylene]-2,4-thiazolidinedione (CA Index name : 329215-18-1), *N*3-[4-(aminosulfonyl)phenyl]-1-(2,6-difluorobenzoyl)- 1*H*-1,2,4-triazole-3,5-diamine (CA Index name : 443797-96-4), 3-(2,3-dihydro-5-benzofuranyl)-1,2,3,4-tetrahydro-2-(2-pyridinyl)-9*H*-pyrrolo[3,4-b]quinolin-9-one, (3R) mono methanesulfonate; 3-(2,3-dihydro-5-benzofuranyl)-1,2,3,4-tetrahydro-2-[5-(2-pyridinyl)-2-pyrimidinyl]-9*H*-pyrrolo [3,4-b]quinolin-9-one, (3R) (CA Index name : 374927-06-7) were chosen as model compounds. The solubility in water of these drugs are reported in Table 3.

**Table 3 :** Solubility in water of model compounds

| Drug | solubility in water* (mg/ml) |
|---|---|
| Risperidone | 0.060 |
| Ketoconazole | 0.010 |
| Indomethacin | 0.010 |
| Hydrocortisone | 0.035 |
| Cyclosporin | 0.001 |
| Amphotericin B | 0.0001 |
| TMC 120 (4-[[4-[(2,4,6-trimethylphenyl)amino]-2-pyrimidinyl]amino]benzonitrile) | < 0.001 |
| TMC 125 (4-[[6-amino-5-bromo-2-[(4-cyanophenyl)amino]-4-pyrimidinyl]oxy]-3,5-dimethylbenzonitrile) | < 0.001 |
| 4-[[4-[[4-(2-cyanoethenyl)-2,6-dimethylphenyl]amino]-2-pyrimidinyl]amino] benzonitrile | 0.00002 |
| 3-[[4-[(2E)-3-(4-fluorophenyl)-2-methyl-2-propenyl]-1-piperazinyl]methyl]-3a,4-dihydro-7,8-dimethoxy-3*H*[1]benzopyrano[4,3-c]isoxazole [(3R,3aS)-rel-(+)] (CA Index name : 452314-01-1) | 0.003 |
| *N*-[[(5S)-3-[4-(2,6-dihydro-2-methylpyrrolo[3,4-c]pyrazol-5(4*H*)-yl)-3-fluorophenyl]-2-oxo-5-oxazolidinyl]methyl]-acetamide (CA Index name: 474016-05-2) | 0.016 |
| (Z) 5-[[3-(5,6,7,8-tetrahydro-3,5,5,8,8-pentamethyl-2-naphthalenyl)-4-(trifluoromethoxy)phenyl]methylene]-2,4-thiazolidinedione (CA Index name: 329215-18-1) | < 0.0005 |
| *N*3-[4-(aminasulfonyl)phenyl]-1-(2,6-difluorobenzoyl)-1*H*-1,2,4-triazole-3,5-diamine (CA Index name : 443797-96-4) | 0.015 |
| 3-(2,3-dihydro-5-benzofuranyl)-1,2,3,4-tetrahydro-2-(2-pyridinyl)-9*H*-pyrrolo[3,4-b] quinolin-9-one, (3R) mono methanesulfonate | 0.190 |
| 3-(2,3-dihydro-5-benzofuranyl)-1,2,3,4-tetrahydro-2-[5-(2-pyridinyl)-2-pyrimidinyl]-9*H*-pyrrolo[3,4-b]quinolin-9-one, (3R) (CA Index name : 374927-06-7) | <0.001 |
| * solubility of the drug in water was measured by mixing appropriate amounts of the drug with water for 24 hours and measuring the absorbance of the solution by UV spectroscopy (Data are expressed as mean values of triplicate sample) | |

To measure the drug loading capacity for ketoconazole, risperidone, hydrocortisone, indomethacin and cyclosporin, an excess of drug was mixed with the copolymer at room temperature for 24 hours on a magnetic stirrer. Water was then added to reach a polymer concentration of 1, 3.15, 10 or 31.5 % w/v (weight/volume). The drug - polymer - water mixture was stirred for 24 hours. The suspension was then filtered through a $0.45 \mu m$ PVDF membrane filter. The filtered solution was immediately diluted to allow the determination of the drug concentration.

Drug concentration was determined by UV spectroscopy (KONTRON Uvikon 940 UV/Visible spectrometer-HP8453 from Hewlett Packard) against a blank containing the same polymer concentration. The filtration of the samples containing the copolymer and the drug through $0.45 \mu m$ PVDF membrane filter (Millipore SLHV025LS) induced no significant decrease of the absorbance.

The solubility of amphotericin B in 1 and 10 % (w/v) aqueous solutions of polymer D4.1 was determined as follows : 100 $\mu$l of a stock solution of amphotericin B (10 mg/ml in dimethylsulfoxide) were placed in a vial and the solvent was allowed to evaporate. Polymer (0.1 g respectively 1 g) was added and mixed for 24 hours. 10 ml of ultrapure water were then added to form the micellar solution and the solution was filtered (0.45 $\mu$m). The amount of amphotericin B was quantified with a UV-visible spectrometer HP8453 after dilution of the solutions with the corresponding polymeric solution to get an absorbance between 0.2 and 0.8.

The solubility of the other model compounds in aqueous solutions of polymer D4.1 was determined by mixing the model compound directly with the polymeric solution for 24 hours at room temperature. Tested polymer concentrations were 1, 5, 10 and 20 % (w/v). The obtained suspension was then filtered through a 0.45$\mu$m PVDF membrane filter. The filtered solution was immediately diluted to allow determination of compound concentration by UV spectroscopy. (A polymer solution of the same concentration as the sample to be analysed was used as blank).

The solubility of risperidone, ketoconazole, hydrocortisone, indomethacin, cyclosporin and amphotericin **B** are reported in Table 4A. Table 4B reports the results obtained for the other tested compounds.

**Table 4A :** Solubility of risperidone, ketoconazole, hydrocortisone, indomethacin, cyclosporin and amphotericin B (mg/ml) in aqueous solutions of diblock copolymers of formula A-B. The solubility was determined in triplicate at room temperature.

| | Polymer concentration (w/v%) | | | |
|---|---|---|---|---|
| **ketoconazole** | **1%** | **3.15%** | **10%** | **31.5%** |
| Polymer | | | | |
| D1.6 | 0.22 | 0.55 | 2.2 | 6.5 |
| D 2 | 0.23 | 0.55 | 1.75 | 5.9 |
| D 4.1 | 0.16 | 0.58 | 1.81 | 4.79 |
| D 4.2 | 0.18 | 0.53 | 1.88 | 5.91 |
| **Risperidone** | | | | |
| Polymer | | | | |
| D1.6 | 0.38 | 0.79 | 2.9 | |
| D 2 | 0.50 | 1.01 | 2.7 | 7.5 |
| D 4.1 | 0.32 | 0.95 | 2.03 | 5.90 |
| D 4.2 | 0.33 | 0.79 | 2.49 | 6.89 |
| D 4.4 | 0.32 | 0.7 | 1.97 | |
| **hydrocortisone** | | | | |
| Polymer | | | | |
| D 4.3 | 0.43 | 0.65 | 1.39 | |
| D 4.4 | 0.42 | 0.56 | 1.47 | |
| **indomethacin** | | | | |
| Polymer | | | | |
| D 4.3 | 0.36 | 1.23 | 3.7 | |
| **cyclosporin** | | | | |
| Polymer | | | | |
| D4.1 | 0.017 | 1.290 | 4.323 | |
| **Amphotericin B** | | | | |
| Polymer | | | | |
| D4.1 | 0.02 | | 0.04 | |

**Table 4B :** Solubility of tested model compounds (mg/ml) in aqueous solutions of diblock copolymer D4.1. The solubility was determined in duplicate at room temperature.

| Compound | D4.1 polymer concentration (w/v %) | Solubility (mg/ml) |
|---|---|---|
| 3-[[4-[(2E)-3-(4-fluorophenyl)-2-methyl-2-propenyl]-1-piperazinyl]methyl]-3a,4-dihydro-7,8-dimethoxy-3*H*-[1]benzopyrano[4,3-c]-isoxazole [(3R,3aS)-rel-(+)] (CA Index name : 452314-01-1) | 1 | 0.200 |
| | 5 | 1.010 |
| | 10 | 1.880 |
| | 20 | 3.860 |
| *N*-[[(5S)-3-[4-(2,6-dihydro-2-methylpyrrolo[3,4-c]pyrazol-5(4*H*)-yl)-3-fluorophenyl]-2-oxo-5-oxazolidinyl]methyl]-acetamide (CA Index name: 474016-05-2) | 1 | 0.020 |
| | 5 | 0.060 |
| | 10 | 0.100 |
| | 20 | 0.190 |
| (Z) 5-[[3-(5,6,7,8-tetrahydro-3,5,5,8,8-pentamethyl-2-naphthalenyl)-4-(trifluoromethoxy)phenyl]methylene]-2,4-thiazolidinedione (CA Index name : 329215-18-1) | 1 | 0.034 |
| | 5 | 0.130 |
| | 10 | 0.350 |
| | 20 | 0.580 |
| *N*3-[4-(aminosulfonyl)phenyl]-1-(2,6-difluorobenzoyl)- 1*H*-1,2,4-triazole-3,5-diamine (CA Index name : 443797-96-4) | 1 | 0.530 |
| | 5 | 2.710 |
| | 10 | 5.760 |
| | 20 | 13.890 |
| 3-(2,3-dihydro-5-benzofuranyl)-1,2,3,4-tetrahydro-2-(2-pyridinyl)-9*H*-pyrrolo[3,4-b]-quinolin-9-one, (3R) mono methanesulfonate | 1 | 4.370 |
| | 5 | 9.830 |
| | 10 | 12.380 |
| | 20 | 13.950 |
| 3-(2,3-dihydro-5-benzofuranyl)-1,2,3,4-tetrahydro-2-[5-(2-pyridinyl)-2-pyrimidinyl]-9*H*-pyrrolo[3,4-b]quinolin-9-one, (3R) (CA Index name: 374927-06-7) | 1 | 0.018 |
| | 5 | 0.081 |
| | 10 | 0.150 |
| | 20 | 0.280 |
| TMC125 | 1 | 0.06 |
| | 5 | 0.3 |
| | 10 | 0.52 |
| | 20 | 0.87 |
| 4-[[4-[[4-(2-cyanoethenyl)-2,6-dimethylphenyl]-amino]-2-pyrimidinyl]amino]benzonitrile | 1 | 0.05 |
| | 5 | 0.12 |
| | 10 | 0.23 |
| | 20 | 0.44 |

Comparison of the values reported in Table 4A and Table 4B with the solubility values in water (Table 3) shows that the copolymeric micellar solutions significantly enhance the water-solubility of the model drugs. For instance, the solubility of hydrocortisone in a solution containing 1 % of copolymer D4.3 is 0.43 mg/ml whereas its solubility in water is 0.035 mg/ml. This means an increase in solubility by a factor of 12. The solubility of indomethacin in a 1 % (w/v) aqueous solution of polymer D4.3 is 0.36 mg/ml. As its solubility in water is 0.01 mg/ ml, the copolymer increased its solubility by a factor of 36.

The results gathered in Table 4A for the copolymer series D4 (D4.1, D4.3, D4.4) point out the reproducibility of the solubilization properties of the same polymer from one synthesis batch to another.

The influence of polymer concentration on the solubility of the model drugs was also assessed. As shown in Tables 4A and 4B, the solubility of almost all drugs increased linearly with the polymer concentration.

Tables 4A and 4B also indicate that reasonable drug contents were obtained without the use of organic solvents. Drug content (% weight/weight) was calculated as follows :

$$\frac{\text{Mass of drug solubilized}}{\text{Mass of polymer}} \times 100$$

The solubility increasing capacity of the diblock copolymers of the present invention (as described above) was compared with that of conventional surfactants and complexing agents such as Tween 20, Tween 80 and cyclodextrin. It was found that for the majority of the tested compounds, the amount of drug solubilized by the copolymers of the invention was at least two times more (usually 20 to 50 times more) compared to the amount solubilized by the conventional solubilizers.

In vitro evaluation of the cytotoxicity of diblock copolymer D4.3

To be used for the encapsulation of drugs in pharmaceutical applications, the diblock copolymers should be non-toxic.

To evaluate cytotoxicty against the intestinal epithelium, a classic MTT test was performed with polymer D4.3 on Caco-2 monolayers.

The MTT test is based on the reduction of MTT (3-(4,5-dimethylthiazol-2-yl)-2,5-diphenyl tetrazolium bromide) to the blue formazan product by mitochondrial enzyme succinate dehydrogenase in viable cells (Mosmann T, J of Immunological Methods, 65, 55-63, 1983). $10^4$ cells per well were incubated in 96 wells for 48 hours at 37°C. Subsequently, the incubation medium was removed and the cells were then incubated for 45 minutes with 180 $\mu$l of the micelle solution in Krebs (0.1 g D4.3/ml) at 37°C. After removal of the solution, 180 $\mu$l of fresh medium (Krebs solution) was added as well as 25 $\mu$l MTT (1g/l in PBS) in each well. After 2 hours of incubation at 37°C, the medium was removed, 25 $\mu$l of glycine buffer and then 100 $\mu$l dimethyl sulfoxide were added per well to dissolve the blue formozan and the optical absorbance read at 490 nm. The MTT test was also performed in the presence of a negative control (medium) and a positive control (Brij 35).

The results obtained for the solutions containing the diblock copolymer were not significantly different from the negative control indicating that the copolymer may be considered as being non-toxic to Caco-2 cells.

The MTT test was also performed with risperidone or ketoconazole loaded D4.3 micelles, on the free drugs and on other copolymers of Table 1. The results obtained were similar to those described above.

In vivo determination of the bioavailability of risperidone encapsulated in copolymer D4.3

As the compositions of the present invention may be used for oral or parenteral administration, it was examined whether orally administered drugs encapsulated in the copolymers of the present invention could go through the intestinal barrier and the blood-brain barrier in vivo and reach their receptors. The model drug used in this study is risperidone, an antipsychotic drug that fixes on D2 dopamine receptors. These receptors are located mainly in the temporal cortex and more precisely in the striatum and in the pituitary gland; the striatum is located at the other side of the encephalic barrier. The test was realized with risperidone carried by micelles made of copolymer D4.3. The principle of the method used is based on the quantification of the receptor occupancy by autoradiography using a [125I] radioligand (Langlois X, Te Riele P., Wintmolders C., Leysen J.E., Jurzak M, J of Pharmacology and Exp Therapeutics, 299, 712-717, 2001).

Male Wistar rats (~200 g) were treated by oral administration of vehicles of 2.5 mg/kg risperidone solubilized in three different vehicles (tartaric acid 0.625 % v/v (as the reference), copolymer D4.3 1 % (w/v) in water and D4.3 10% (w/v) in water). The animals were sacrificed by decapitation 2 hours after administration.

After decapitation, the brains were immediately removed from the skull and rapidly frozen in dry ice (cooled 2-Methylbutane (-40°C)) for approximately 2 minutes. The brains were stored at -20°C for at least 24 hours before sectioning. Twenty $\mu$m-thick frontal sections were cut using a Leica CM 3050 cryostat-microtome and thaw-mounted on adhesive microscope slides. Three adjacent brain slices from the same animal were collected per slide. Two brain slices were used to measure the total binding and the third one was evaluated for non-specific binding. The sections were kept at -20°C for at least 24 hours before being incubated with the radioligand ([125I] Iodosulpride, Amersham).

The occupancy of D2 receptors by risperidone was measured in the striatum and the pituitary gland of each individual rat. The following general procedure was applied: after thawing, sections were dried under a stream of cold air. The sections were not washed prior to incubation, in order to avoid dissociation of the drug-receptor complex. Brain and pituitary gland sections from drug-treated and vehicle-treated animals were incubated in parallel with the radioligand and the 10 minutes incubation time was rigorously controlled. After the incubation, the excess of radioligand was washed off in ice-cold buffer, followed by a quick rinse in cold distilled water. The sections were then dried under a stream of cold air, placed in a light-tight cassette and covered with Ektascan GRL films (Kodak). After the exposure time, the films were developed in a Kodak X-Omat processor.

Autoradiograms were quantified using an MCID (MicroComputer Imaging Device) M1 image analyzer (Imaging Research, St-Catharines, Ontario, Canada). Optical densities were transformed into levels of bound radioactivity after calibration of the image analyzer using gray-values generated by co-exposure with the tissue sections, of commercially available polymer standards ([125I] Micro-scales, Amersham). Specific binding was calculated as the difference between total binding and non-specific binding. *Ex vivo* receptor labeling by the radioligand in brain sections of drug-treated animals was expressed as the percentage of receptor labeling in corresponding brain sections of vehicle-treated animals. Since only unoccupied receptors remain available for the radioligand, *ex-vivo* receptor labeling is inversely proportional to the receptor occupancy by the *in vivo* administered drug. Percentages,of receptor occupancy by the drug administered to the animal correspond to 100% minus the percentage of receptors labeled in the treated animal.

To reach the pituitary gland, the drug has to go across the intestinal barrier and be carried by the blood to the gland. To reach the striatum, the drug has, in addition, to pass across the blood-brain barrier. Risperidone was administered in a reference vehicle (tartaric acid) or after solubilization in an aqueous solution containing 1% or 10% of copolymer D4.3.

The obtained results indicated that risperidone transported by the copolymer solutions or by the reference solution could reach the D2 receptors located in the pituitary gland.

To reach the pituitary gland, the drug has to go across the intestinal barrier and be carried by the blood to the gland.

The obtained results also indicated that risperidone transported by the copolymer solutions or the reference solution could reach the D2 receptors located in the striatum. To reach the striatum, the drug has to go across the intestinal barrier and, in addition, has to pass across the blood-brain barrier.

These results indicate that the oral administration of risperidone by the self-emulsifying copolymer D4.3 did not affect the passage of this drug through the intestinal barrier. They also indicate that risperidone, whether encapsulated or not in the micelles, passed through the blood-brain barrier and reached the target receptors.

The above experiment was also performed in function of time for the reference solution and for the D4.3 10% (w/v) aqueous solution. Therefore, animals were sacrificed by decapitation at 10 minutes, 30 minutes, 1 hour, 2 hours, 4 hours, 8 hours, 16 hours and 24 hours after administration of the risperidone formulations. Both formulations showed a similar D2 receptor occupancy during the first 8 hours. During this experiment, the usual pharmacokinetic parameters of the active metabolite following oral administration of the two risperidone solutions were determined by LCMS analysis of the plasma samples. It was found that the AUC of the drug was slightly higher when the drug was solubilized in the micellar solution of the invention. The longer half-life and the lower Cmax which were determined for the micellar solution compared to the reference solution, suggest that the polymeric micelles may provide a sustained release of the drug. This finding confirmed the results of an *in vitro* release study of risperidone from a micellar solution of the present invention. The *in vitro* release study was performed by dialysing a micellar solution of the invention containing $C^{14}$-radioactive risperidone against water and determining the radioactivity by liquid scintillation counting of samples taken from the water as a function of time.

Effect of encapsulation of Amphotericin B in micelles of the invention on hemolysis induced by the drug

Amphotericin B is a drug that is used to treat systemic mycosis. It is poorly soluble in water unless it is formulated with desoxycholate (Fungizone®). It is known that amphotericin B induces hemolysis.

In order to determine the effect of the encapsulation of amphotericin B on the hemolysis induced by the drug, hemolysis induced by different concentrations of the drug (0, 3, 6, 12, 18, 24 μg/ml) formulated as a water-soluble formulation and encapsulated in a 10 % micellar solution of polymer D4.3 was compared.
The samples were prepared as follow :

- Water-soluble formulation: 50 mg Fungizone® (= amphotericin B 50mg + sodium desoxycholate 41mg + phosphate disodium and phosphate monosodic 20.2g) was solubilized in 10 ml water for injection (Mini-Plasco®). The different concentrations were obtained by dilution of this solution with isotonic PBS (pH 7,41).

- Amphotericin B encapsulated in a 10 % micellar solution of polymer D4.3. The solutions were prepared by mixing the micellar solution prepared in isotonic PBS for one night to amphotericin B (Sigma-Aldrich, cell culture tested) which was obtained from a solution in dimethylsulfoxide after evaporation.

Red blood cells of 3 rats were obtained by intracardiac punction. The blood was then centrifuged (2000 rounds per minute-10minutes-25°C) and the supernatant eliminated. Red blood cells were diluted with isotonic PBS in order to obtain an absorbance between 0.8 and 1 at a 100 % hemolysis. 2.5ml of the red blood cells solution were incubated for 30 minutes at 37°C under agitation with 2.5ml of the different solutions. After centrifugation (2000rpm - 10min - 25°C), the absorbance was measured at 576 nm. Total hemolysis was provoked by a hypoosmotic aqueous solution containing 24$\mu$g/ml Fungizone®. (Tasset et al, 1990).
The hemolysis % was determined by the following formula:

$$\text{Hemolysis } \% = 100 \ (abs-abs0)/(abs100-abs0)$$

Where Abs = absorbance of the sample
Abs 100 = absorbance at 100 % hemolysis
Abs 0 = absorbance at 0% hemolysis (Lavasanifar et al, 2002)

It was found that when amphotericin is encapsulated in the micellar solution, hemolysis was limited to less than 5 % up to an amphotericin B concentration of 12 $\mu$g/ml, whereas Fungizone®, the water soluble formulation of amphotericin B, induced already 25% hemolysis at a concentration of amphotericin B as low as 6$\mu$g/ml. Thus, micellar encapsulation in the micelles of the present invention decreases the toxic effect of amphotericin B.

From the above-indicated experiments, it can be concluded that the compositions of the present invention are promising micellar delivery systems for the delivery of poorly water-soluble drugs, especially for oral or parenteral administration. Compared to existing diblock copolymers that form polymeric micelles, the present copolymers have the advantage to spontaneously form micelles in water, the micelles being stable in physiological conditions. Neither extensive heat or organic solvents, complex or time consuming manufacturing procedures are needed to produce micelles or to incorporate drugs into the micelles. The CMC of the present copolymers is sufficiently low to assume that the copolymer concentration in the gastro-intestinal tract or blood will remain above the CMC after administration. The synthesis of the copolymers is reproducible. The solubility of poorly water soluble drugs in aqueous solutions of the present copolymers is increased when compared to pure water. The copolymers are non-toxic to Caco-2 cells. Experiments with a model drug revealed that the copolymers have no significant impact on the bioavailability of the drug. Encapsulation of drug in polymeric micelles of the invention may result in slow, controlled, sustained release of drug. Encapsulation into the present micelles may also reduce the toxicity of the encapsulated drug.

**Claims**

1. A diblock copolymer of formula A-B wherein
   polymer block A represents a linear pharmaceutically acceptable hydrophilic polymer with a molecular weight < 1,000, and
   polymer block B represents a polymer comprising at least two different monomers selected from glycolic acid, propiolactone, γ-butyrolactone, δ-valerolactone, γ-valerolactone, ε-caprolactone, trimethylene carbonate, p-dioxanone, tetramethylene carbonate, ε-lactone, 1,5-dioxepan-2-one **characterized in that** the diblock copolymer is liquid at a temperature below 50°C.

2. A diblock copolymer according to claim 1 wherein polymer block B represents a
   polymer comprising monomers selected from glycolic acid, propiolactone, γ-butyrolactone, δ-valerolactone, ε-caprolactone, trimethylene carbonate,
   p-dioxanone, tetramethylene carbonate, ε-lactone, 1,5-dioxepan-2-one or mixtures thereof.

3. A diblock copolymer according to claim 1 wherein polymer block B represents a polymer comprising monomers of trimethylene carbonate and monomers selected from glycolic acid, propiolactone, γ-butyrolactone, δ-valerolactone, γ-valerolactone, ε-caprolactone, p-dioxanone, tetramethylene carbonate, ε-lactone, 1,5-dioxepan-2-one or mixtures thereof.

4. A diblock copolymer according to claim 3 wherein polymer block B represents a polymer comprising monomers of trimethylene carbonate and monomers selected from glycolic acid, propiolactone, $\gamma$-butyrolactone, $\delta$-valerolactone, $\varepsilon$-caprolactone, p-dioxanone, tetramethylene carbonate, $\varepsilon$-lactone, 1,5-dioxepan-2-one or mixtures thereof.

5. A diblock copolymer according to claim 1 wherein polymer block B represents a polymer comprising monomers selected from propiolactone, $\gamma$-butyrolactone, $\delta$-valerolactone, $\gamma$-valerolactone, $\varepsilon$-caprolactone, trimethylene carbonate, p-dioxanone, tetramethylene carbonate, $\varepsilon$-lactone, 1,5-dioxepan-2-one.

6. A diblock copolymer according to claim 5 wherein polymer block B comprises two different monomers selected from propiolactone, $\gamma$-butyrolactone, $\delta$-valerolactone, $\gamma$-valerolactone, $\varepsilon$-caprolactone, trimethylene carbonate, p-dioxanone, tetramethylene carbonate, $\varepsilon$-lactone, 1,5-dioxepan-2-one.

7. A diblock copolymer according to claim 6 wherein polymer block B comprises monomers selected from $\varepsilon$-caprolactone and trimethylene carbonate.

8. A diblock copolymer of formula A-B wherein
polymer block A represents a linear pharmaceutically acceptable hydrophilic polymer with a molecular weight < 1,000, and
polymer block B represents a polymer comprising monomers of trimethylene carbonate and monomers selected from glycolic acid, propiolactone, $\gamma$-butyrolactone, $\delta$-valerolactone, $\varepsilon$-caprolactone, trimethylene carbonate, p-dioxanone, tetramethylene carbonate, $\varepsilon$-lactone, 1,5-dioxepan-2-one or mixtures thereof.
**characterized in that** the diblock copolymer is liquid at a temperature below 50°C.

9. A diblock copolymer according to any one of claims 1 to 8 wherein polymer block A represents poly($C_{1-20}$alkylene oxide) or a derivative thereof.

10. A diblock copolymer according to claim 9 wherein the poly($C_{1-20}$alkylene oxide) or the derivative thereof is poly(ethylene glycol) or a derivative thereof, in particular poly(ethylene glycol) monomethylether.

11. A diblock copolymer according to claim 10 wherein the poly(ethylene glycol) or a derivative thereof has a molecular weight ranging from > 350 to ≤ 750.

12. A diblock copolymer according to claim 11 wherein the poly(ethylene glycol) or the derivative thereof has a molecular weight of 750.

13. A diblock copolymer according to any one of claims 1 to 12 having a molecular weight ranging from 2,000 to 10,000.

14. A diblock copolymer according to claim 13 having a molecular weight ranging from 2,000 to 8,000.

15. A diblock copolymer according to claim 14 having a molecular weight ranging from 2,500 to 7,000.

16. A diblock copolymer according to any one of claims 1 to 15 being a liquid at room temperature or at 37°C.

17. A composition comprising an active ingredient and one or more diblock copolymers of formula A-B according to any one of claims 1 to 16 **characterized in that** the composition is liquid below 50°C.

18. A composition according to claim 17 wherein the composition is non-aqueous.

19. A pharmaceutical dosage form comprising a therapeutically effective amount of a composition according to claim 17 or 18.

20. A pharmaceutical dosage form according to claim 19 **characterized in that** the dosage form is suitable for oral administration.

21. A pharmaceutical dosage form according to claim 19 **characterized in that** the dosage form is suitable for parenteral administration.

22. A pharmaceutical dosage form according to any one of claims 19 to 21 wherein the dosage form is an aqueous solution.

23. A process to prepare an aqueous solution comprising an active ingredient and one or more diblock copolymers of formula A-B according to any one of claims 1 to 16 **characterized by** mixing the active ingredient with the one or more liquid copolymers, i.e. at a temperature below 50°C, followed by addition of water while stirring.

24. A process to prepare an aqueous solution comprising an active ingredient and one or more diblock copolymers of formula A-B according to any one of claims 1 to 16 **characterized by**

   a) mixing the one or more copolymers with water at a temperature below 50°C, followed by
   b) the addition of the active ingredient to the aqueous polymeric solution obtained under a) while stirring.

25. Use of a composition according to claim 17 or 18 for the manufacture of a pharmaceutical dosage form for oral administration to a human or non-human animal in need of treatment.

26. Use of a composition according to claim 17 or 18 for the manufacture of a pharmaceutical dosage form for parenteral administration to a human or non-human animal in need of treatment.

27. A pharmaceutical package suitable for commercial sale comprising a container, a pharmaceutical dosage form according to any one of claims 19 to 22, and associated with said package written matter.


**Patentansprüche**

1. Diblock-Copolymer der Formel A-B, wobei Polymerblock A für ein geradkettiges, pharmazeutisch unbedenkliches, hydrophiles Polymer mit einem Molekulargewicht < 1000 steht und Polymerblock B für ein Polymer steht, das wenigstens zwei verschiedene Monomere ausgewählt aus Glykolsäure, Propiolacton, γ-Butyrolacton, δ-Valerolacton, γ-Valerolacton, ε-Caprolacton, Trimethylencarbonat, p-Dioxanon, Tetramethylencarbonat, ε-Lacton, 1,5-Dioxepan-2-on enthält, **dadurch gekennzeichnet, daß** das Diblock-Copolymer bei einer Temperatur unterhalb von 50°C flüssig ist.

2. Diblock-Copolymer nach Anspruch 1, wobei Polymerblock B für ein Polymer steht, das Monomere ausgewählt aus Glykolsäure, Propiolacton, γ-Butyrolacton, δ-Valerolacton, ε-Caprolacton, Trimethylencarbonat, p-Dioxanon, Tetramethylencarbonat, ε-Lacton, 1,5-Dioxepan-2-on und Mischungen davon enthält.

3. Diblock-Copolymer nach Anspruch 1, wobei Polymerblock B für ein Polymer steht, das Monomere von Trimethylencarbonat und Monomere ausgewählt aus Glykolsäure, Propiolacton, γ-Butyrolacton, δ-Valerolacton, γ-Valerolacton, ε-Caprolacton, p-Dioxanon, Tetramethylencarbonat, ε-Lacton, 1,5-Dioxepan-2-on und Mischungen davon enthält.

4. Diblock-Copolymer nach Anspruch 3, wobei Polymerblock B für ein Polymer steht, das Monomere von Trimethylencarbonat und Monomere ausgewählt aus Glykolsäure, Propiolacton, γ-Butyrolacton, δ-Valerolacton, ε-Caprolacton, p-Dioxanon, Tetramethylencarbonat, ε-Lacton, 1,5-Dioxepan-2-on und Mischungen davon enthält.

5. Diblock-Copolymer nach Anspruch 1, wobei Polymerblock B für ein Polymer steht, das Monomere ausgewählt aus Propiolacton, γ-Butyrolacton, δ-Valerolacton, γ-Valerolacton, ε-Caprolacton, Trimethylencarbonat, p-Dioxanon, Tetramethylencarbonat, ε-Lacton und 1,5-Dioxepan-2-on enthält.

6. Diblock-Copolymer nach Anspruch 5, wobei Polymerblock B zwei verschiedene Monomere ausgewählt aus Propiolacton, γ-Butyrolacton, δ-Valerolacton, γ-Valerolacton, ε-Caprolacton, Trimethylencarbonat, p-Dioxanon, Tetramethylencarbonat, ε-Lacton und 1,5-Dioxepan-2-on enthält.

7. Diblock-Copolymer nach Anspruch 6, wobei Polymerblock B Monomere ausgewählt aus ε-Caprolacton und Trimethylencarbonat enthält.

8. Diblock-Copolymer der Formel A-B, wobei Polymerblock A für ein geradkettiges, pharmazeutisch unbedenkliches, hydrophiles Polymer mit einem Molekulargewicht < 1000 steht und Polymerblock B für ein Polymer steht, das Monomere von Trimethylencarbonat und Monomere ausgewählt aus Glykolsäure, Propiolacton, γ-Butyrolacton, δ-Valerolacton, ε-Caprolacton, Trimethylencarbonat, p-Dioxanon, Tetramethylencarbonat, ε-Lacton, 1,5-Dioxepan-2-on und Mischungen davon enthält, **dadurch gekennzeichnet, daß** das Diblock-Copolymer bei einer Temperatur unterhalb von 50°C flüssig ist.

9. Diblock-Copolymer nach einem der Ansprüche 1-8, wobei Polymerblock A für Poly($C_{1-20}$-alkylenoxid) oder ein Derivat davon steht.

10. Diblock-Copolymer nach Anspruch 9, wobei es sich bei dem Poly($C_{1-20}$-alkylenoxid) bzw. dem Derivat davon um Poly(ethylenglykol) oder ein Derivat davon, insbesondere Poly(ethylenglykol)monomethylether handelt.

11. Diblock-Copolymer nach Anspruch 10, wobei das Poly(ethylenglykol) bzw. ein Derivat davon ein Molekulargewicht im Bereich von > 350 bis ≤ 750 aufweist.

12. Diblock-Copolymer nach Anspruch 11, wobei das Poly(ethylenglykol) bzw. das Derivat davon ein Molekulargewicht von 750 aufweist.

13. Diblock-Copolymer nach einem der Ansprüche 1 bis 12 mit einem Molekulargewicht im Bereich von 2000 bis 10000.

14. Diblock-Copolymer nach Anspruch 13 mit einem Molekulargewicht im Bereich von 2000 bis 8000.

15. Diblock-Copolymer nach Anspruch 14 mit einem Molekulargewicht im Bereich von 2500 bis 7000.

16. Diblock-Copolymer nach einem der Ansprüche 1 bis 15, das bei Raumtemperatur oder bei 37°C flüssig ist.

17. Zusammensetzung, enthaltend einen Wirkstoff und ein oder mehrere Diblock-Copolymere der Formel A-B nach einem der Ansprüche 1 bis 16, **dadurch gekennzeichnet, daß** die Zusammensetzung unterhalb von 50°C flüssig ist.

18. Zusammensetzung nach Anspruch 17, wobei die Zusammensetzung nicht-wäßrig ist.

19. Pharmazeutische Dosierungsform, enthaltend eine therapeutisch wirksame Menge einer Zusammensetzung nach Anspruch 17 oder 18.

20. Pharmazeutische Dosierungsform nach Anspruch 19, **dadurch gekennzeichnet, daß** sich die Dosierungsform für die orale Verabreichung eignet.

21. Pharmazeutische Dosierungsform nach Anspruch 19, **dadurch gekennzeichnet, daß** sich die Dosierungsform für die parenterale Verabreichung eignet.

22. Pharmazeutische Dosierungsform nach einem der Ansprüche 19 bis 21, wobei es sich bei der Dosierungsform um eine wäßrige Lösung handelt.

23. Verfahren zur Herstellung einer wäßrigen Lösung enthaltend einen Wirkstoff und ein oder mehrere Diblock-Copolymere der Formel A-B nach einem der Ansprüche 1 bis 16, **dadurch gekennzeichnet, daß** man den Wirkstoff mit dem einen oder den mehreren flüssigen Copolymeren, d.h. bei einer Temperatur unterhalb von 50°C, mischt, und anschließend unter Rühren Wasser zusetzt..

24. Verfahren zur Herstellung einer wäßrigen Lösung enthaltend einen Wirkstoff und ein oder mehrere Diblock-Copolymere der Formel A-B nach einem der Ansprüche 1 bis 16, **dadurch gekennzeichnet, daß** man

    a) das eine oder die mehreren Copolymere bei einer Temperatur unterhalb 50°C mit Wasser mischt und anschließend
    b) unter Rühren den Wirkstoff zu der unter a) erhaltenen wäßrigen polymeren Lösung gibt.

25. Verwendung einer Zusammensetzung nach Anspruch 17 oder 18 zur Herstellung einer pharmazeutischen Dosierungsform für die orale Verabreichung an ein behandlungsbedürftiges menschliches oder nicht-menschliches Tier.

26. Verwendung einer Zusammensetzung nach Anspruch 17 oder 18 zur Herstellung einer pharmazeutischen Dosierungsform für die parenterale Verabreichung an ein behandlungsbedürftiges menschliches oder nicht-menschliches Tier.

27. Pharmazeutische Packung, geeignet für den kommerziellen Verkauf, umfassend ein Behältnis, eine pharmazeutische Dosierungsform nach einem der Ansprüche 19 bis 22 und mit der Packung assoziiertes Schriftgut.

**Revendications**

1. Copolymère à deux blocs de formule A-B dans lequel le bloc A du polymère représente un polymère hydrophile linéaire pharmaceutiquement acceptable ayant une masse molaire < 1 000, et
le bloc B du polymère représente un polymère comprenant au moins deux monomères différents choisis parmi l'acide glycolique, la propiolactone, la γ-butyrolactone, la δ-valérolactone, la γ-valérolactone, l'ε-caprolactone, le carbonate de triméthylène, la p-dioxanone, le carbonate de tétraméthylène, l'ε-lactone, la 1,5-dioxépan-2-one, lequel copolymère à deux blocs étant **caractérisé en ce qu'**il est liquide à une température inférieure à 50°C.

2. Copolymère à deux blocs selon la revendication 1 dans lequel le bloc B du polymère représente un polymère comprenant des monomères choisis parmi l'acide glycolique, la propiolactone, la γ-butyrolactone, la δ-valérolactone, l'ε-caprolactone, le carbonate de triméthylène, la p-dioxanone, le carbonate de tétraméthylène, l'ε-lactone, la 1,5-dioxépan-2-one ou leurs mélanges.

3. Copolymère à deux blocs selon la revendication 1 dans lequel le bloc B du polymère représente un polymère comprenant des monomères de carbonate de triméthylène et des monomères choisis parmi l'acide glycolique, la propiolactone, la γ-butyrolactone, la δ-valérolactone, la γ-valérolactone, l'ε-caprolactone, la p-dioxanone, le carbonate de tétraméthylène, l'ε-lactone, la 1,5-dioxépan-2-one ou leurs mélanges.

4. Copolymère à deux blocs selon la revendication 3 dans lequel le bloc B du polymère représente un polymère comprenant des monomères de carbonate de triméthylène et des monomères choisis parmi l'acide glycolique, la propiolactone, la γ-butyrolactone, la δ-valérolactone, l'ε-caprolactone, la p-dioxanone, le carbonate de tétraméthylène, l'ε-lactone, la 1,5-dioxépan-2-one ou leurs mélanges.

5. Copolymère à deux blocs selon la revendication 1 dans lequel le bloc B du polymère représente un polymère comprenant des monomères choisis parmi la propiolactone, la γ-butyrolactone, la δ-valérolactone, la γ-valérolactone, l'ε-caprolactone, le carbonate de triméthylène, la p-dioxanone, le carbonate de tétraméthylène, l'ε-lactone, la 1,5-dioxépan-2-one.

6. Copolymère à deux blocs selon la revendication 5 dans lequel le bloc B du polymère comprend deux monomères différents choisis parmi la propiolactone, la γ-butyrolactone, la δ-valérolactone, la γ-valérolactone, l'ε-caprolactone, le carbonate de triméthylène, la p-dioxanone, le carbonate de tétraméthylène, l'ε-lactone, la 1,5-dioxépan-2-one.

7. Copolymère à deux blocs selon la revendication 6 dans lequel le bloc B du polymère comprend des monomères choisis parmi l'ε-caprolactone et le carbonate de triméthylène.

8. Copolymère à deux blocs de formule A-B dans lequel le bloc A du polymère représente un polymère hydrophile linéaire pharmaceutiquement acceptable ayant une masse molaire < 1 000, et
le bloc B du polymère représente un polymère comprenant des monomères de carbonate de triméthylène et des monomères choisis parmi l'acide glycolique, la propiolactone, la γ-butyrolactone, la δ-valérolactone, l'ε-caprolactone, le carbonate de triméthylène, la p-dioxanone, le carbonate de tétraméthylène, l'ε-lactone, la 1,5-dioxépan-2-one ou leurs mélanges,
lequel copolymère à deux blocs étant **caractérisé en ce qu'**il est liquide à une température inférieure à 50°C.

9. Copolymère à deux blocs selon l'une quelconque des revendications 1 à 8 dans lequel le bloc A du polymère représente un poly(oxyde d'alkylène en $C_{1-20}$) ou un de ses dérivés.

10. Copolymère à deux blocs selon la revendication 9 dans lequel le poly(oxyde d'alkylène en $C_{1-20}$) ou son dérivé est le poly(éthylèneglycol) ou un de ses dérivés, en particulier l'éther monométhylique de poly-(éthylèneglycol).

11. Copolymère à deux blocs selon la revendication 10 dans lequel le poly(éthylèneglycol) ou un de ses dérivés a une masse molaire qui s'échelonne de > 350 à ≤ 750.

**12.** Copolymère à deux blocs selon la revendication 11 dans lequel le poly(éthylèneglycol) ou son dérivé a une masse molaire de 750.

**13.** Copolymère à deux blocs selon l'une quelconque des revendications 1 à 12 ayant une masse molaire qui s'échelonne de 2 000 à 10 000.

**14.** Copolymère à deux blocs selon la revendication 13 ayant une masse molaire qui s'échelonne de 2 000 à 8 000.

**15.** Copolymère à deux blocs selon la revendication 14 ayant une masse molaire qui s'échelonne de 2 500 à 7 000.

**16.** Copolymère à deux blocs selon l'une quelconque des revendications 1 à 15 qui est un liquide à la température ambiante ou à 37°C.

**17.** Composition comprenant un ingrédient actif et un ou plusieurs copolymères à deux blocs de formule A-B selon l'une quelconque des revendications 1 à 16, laquelle composition étant **caractérisée en ce qu'**elle est liquide au-dessous de 50°C.

**18.** Composition selon la revendication 17, laquelle composition étant non aqueuse.

**19.** Forme galénique comprenant une quantité efficace sur le plan thérapeutique d'une composition selon la revendication 17 ou 18.

**20.** Forme galénique selon la revendication 19 **caractérisée en ce qu'**elle convient pour l'administration orale.

**21.** Forme galénique selon la revendication 19 **caractérisée en ce qu'**elle convient pour l'administration parentérale.

**22.** Forme galénique selon l'une quelconque des revendications 19 à 21, laquelle forme galénique étant une solution aqueuse.

**23.** Procédé de préparation d'une solution aqueuse comprenant un ingrédient actif et un ou plusieurs copolymères à deux blocs de formule A-B selon l'une quelconque des revendications 1 à 16 **caractérisé en ce qu'**il consiste à mélanger l'ingrédient actif avec le ou les copolymères liquides, c'est-à-dire à une température inférieure à 50°C, puis à ajouter de l'eau tout en agitant.

**24.** Procédé de préparation d'une solution aqueuse comprenant un ingrédient actif et un ou plusieurs copolymères à deux blocs de formule A-B selon l'une quelconque des revendications 1 à 16, **caractérisé en ce qu'**il consiste à

a) mélanger le ou les copolymères avec de l'eau à une température inférieure à 50°C, et ensuite
b) ajouter l'ingrédient actif à la solution polymère aqueuse obtenue en a) tout en agitant.

**25.** Utilisation d'une composition selon la revendication 17 ou 18 pour fabriquer une forme galénique destinée à l'administration orale à un être humain ou à un animal non humain ayant besoin d'un traitement.

**26.** Utilisation d'une composition selon la revendication 17 ou 18 pour fabriquer une forme galénique destinée à l'administration parentérale à un être humain ou à un animal non humain ayant besoin d'un traitement.

**27.** Conditionnement pharmaceutique approprié à la vente dans le commerce comprenant un récipient, une forme galénique selon l'une quelconque des revendications 19 à 22 et, accompagnant ledit conditionnement, une notice écrite.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0166596 B
- EP 0711794 B
- EP 0411545 B
- US 6322805 B
- US 5653992 A
- US 5631015 A, Bezwada

### Non-patent literature cited in the description

- **DANIELSSON ; LINDMAN.** *Colloid Surf,* 1981, vol. 3, 391
- **HAGAN et al.** *Langmuir,* 1996, vol. 12, 2153-2161
- **ZHANG et al.** *Int. J. Pharm,* 1996, vol. 132, 195-206
- **MATSUDA et al.** *Macromolecules,* 2000, vol. 33, 795-800
- Protective Groups in Organic Chemistry. Plenum Press, 1973
- Protective Groups in Organic Synthesis. T W Greene & P G M Wutz, Wiley Interscience, 1991
- **MOSMANN T.** *J of Immunological Methods,* 1983, vol. 65, 55-63
- **LANGLOIS X ; TE RIELE P ; WINTMOLDERS C ; LEYSEN J.E ; JURZAK M.** *J of Pharmacology and Exp Therapeutics,* 2001, vol. 299, 712-717